# EUROPEAN PATENT APPLICATION

(11) **EP 1 623 695 A1**
(43) Date of publication of application: **08.02.2006**
(21) Application number: 04732782.0
(22) Date of filing: 13.05.2004
(51) Int. Cl.: A61K 8/18

(54) **OIL-IN-WATER EMULSION COSMETIC**

(30) Priority: 13.05.2003 JP 2003135191
(71) Applicant: The Nisshin OilliO Group, Ltd., Tokyo 104-8285 (JP)
(72) Inventor: GOTOU, Naoki, c/o The Nisshin Oillio Group, Ltd., Yokohama-shi, Kanagawa 2358558 (JP); EHARA, Taro, c/o The Nisshin Oillio Group, Ltd., Yokohama-shi, Kanagawa 2358558 (JP); MORI, Takahiro, c/o The Nisshin Oillio Group, Ltd., Yokohama-shi, Kanagawa 23585 (JP)
(74) Representative: Zimmermann, Gerd Heinrich
(86) International application number: PCT/JP2004/006786
(87) International publication number: WO 2004/100917

(57) **Abstract**

The present invention relates to an oil-in-water emulsion type cosmetic preparation that can offer excellent skin feel (non-sticky feel and long-lasting moisturizing feel) and exhibit excellent long-term emulsion stability, containing (A) one or more members selected from the group consisting of ester compounds which are reaction products of erythritol and/or erythritol condensate with a fatty acid(s); polycondensates of erythritol and/or erythritol condensate, the above-mentioned ester compound(s) and a polycarboxylic acid(s); polycondensates of a fatty acid(s) with a polycondensate(s) of erythritol and/or erythritol condensate with a polycarboxylic acid(s); and polycondensates of erythritol and/or erythritol condensate, a fatty acid(s) and a polycarboxylic acid(s); (B) a nonionic surfactant and/or an ionic surfactant with an HLB of 10 or more; and (C) a water-base ingredient.

## Description

### Background of the Invention

The present invention relates to an oil-in-water emulsion type cosmetic preparation, and more particularly to an oil-in-water emulsion type cosmetic preparation that can meet both requirements of a non-sticky feel and a long-lasting moisturizing feel and exhibit excellent emulsion stability.

Conventionally, the oil-in-water emulsion type cosmetic preparation has an effect of controlling the balance between the water content and the oily content in the skin when applied to the skin, to keep the skin in good condition by the action of ingredients blended in the emulsion, such as an aqueous ingredient, an oily base, a moisturizing agent, a beauty ingredient and the like. Extensive investigations have been made to additionally obtain the benefits with respect to the preservation stability, skin feel, appearance and the like by selecting the ingredients to be blended into the oil-in-water emulsion type cosmetic preparation (as disclosed in, for example, Japanese Patent No. 2657219 and Japanese Patent Unexamined Publication (JP Kokai) No. 2001-39827 and No. 2001-354510).

### Disclosure of Invention

Although various benefits have been hitherto discussed, the benefit for moisturizing feel from the oil-in-water emulsion type cosmetic preparation is poorer than that from other forms containing an oily base, for example, water-in-oil emulsion type cosmetic preparation and oil-based cosmetic preparation. Therefore, there is an increasing demand for development of an oil-in-water emulsion type cosmetic preparation that can give a non-sticky feel, which is one of the features peculiar to the oil-in-water-emulsion type cosmetic preparation, and at the same time, spread smoothly to ensure the feeling realized by the use of it (good feeling to skin) when applied, offer a long-lasting moisturizing feel and exhibit excellent emulsion stability for an extended period of time.

The inventors of the present invention have studied intensively to solve the above-mentioned problems. As a result of the study, it has been found that an oil-in-water emulsion type cosmetic preparation which can offer improved long-lasting moisturizing feel, while maintaining a non-sticky feel and excellent usability, and exhibit excellent emulsification of the oily base to improve the long-term stability, by blending a particular erythritol and/or erythritol condensate derivative, a nonionic surfactant with an HLB of 10 or more and/or ionic surfactant with an HLB of 10 or more, and a water-base ingredient into the formulation. The present invention has been thus accomplished.

Namely, the present invention provides an oil-in-water emulsion type cosmetic preparation comprising the following components:
(A) one or more members selected from the group consisting of ester compounds represented by the following formula (I) and/or formula (II) which are reaction products of erythritol and/or erythritol condensate with a fatty acid(s); polycondensates of erythritol and/or erythritol condensate, the above-mentioned ester compound(s) and a polycarboxylic acid(s); polycondensates of a fatty acid(s) with a polycondensate(s) of erythritol and/or erythritol condensate with a polycarboxylic acid(s); and polycondensates of erythritol and/or erythritol condensate, a fatty acid(s) and a polycarboxylic acid(s), 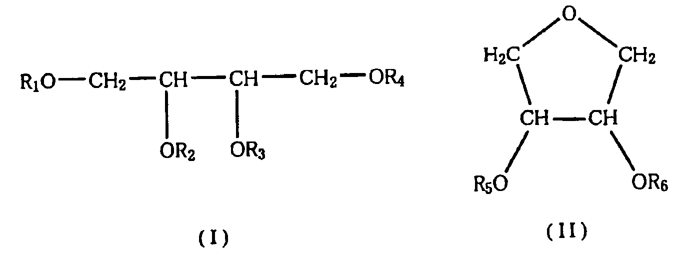
   wherein R₁ to R₄ are each independently hydrogen atom, a fatty acid residue or a polycarboxylic acid residue, and R₅ and R₆ are each independently hydrogen atom, a fatty acid residue or a polycarboxylic acid residue, provided that all of R₁ to R₄ do not represent hydrogen atom at the same time, and both of R₅ and R₆ do not represent hydrogen atom at the same time;
(B) a nonionic surfactant with an HLB of 10 or more and/or an ionic surfactant with an HLB of 10 or more; and
(C) a water-base ingredient.

The above-mentioned polycondensate of fatty acid with the polycondensate of erythritol and/or erythritol condensate with polycarboxylic acid indicates a polycondensate obtainable by subjecting a fatty acid and a polycondensate of erythritol and/or erythritol condensate with a polycarboxylic acid to an esterification reaction.

The present invention also provides the oil-in-water emulsion type cosmetic preparation comprising the components (A), (B) and (C), wherein, for the component (A), at least one of R₁ to R₄ in the formula (I) is hydrogen atom.

The present invention also provides the oil-in-water emulsion type cosmetic preparation comprising the components (A), (B) and (C), wherein, for the component (A), at least one of R₅ or R₆ in the formula (II) is hydrogen atom.

The present invention also provides the oil-in-water emulsion type cosmetic preparation comprising the components (A), (B) and (C), wherein the component (A) is contained in an amount of 0.1 to 30% by mass, the component (B) is contained in an amount of 0.01 to 10% by mass, and the component (C) is contained in an amount of 40 to 95% by mass.

Further, the present invention provides the oil-in-water emulsion type cosmetic preparation comprising the components (A), (B) and (C), wherein the component (A) has a hydroxyl value (OHV) of 10 to 150.

### Best Mode for Carrying out the Invention

The component (A) used in the oil-in-water emulsion type cosmetic preparation of the present invention is one or more members selected from the group consisting of ester compounds of the following formula (I) and/or formula (II) which are reaction products of erythritol and/or erythritol condensate with a fatty acid(s); polycondensates of erythritol and/or erythritol condensate, the above-mentioned ester compound(s) and a polycarboxylic acid(s); polycondensates of a fatty acid(s) with a polycondensate(s) of erythritol and/or erythritol condensate with a polycarboxylic acid(s); and polycondensate(s) of erythritol and/or erythritol condensate, a fatty acid(s) and a polycarboxylic acid(s), 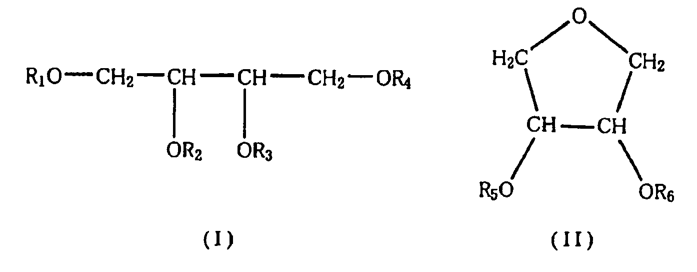
wherein R₁ to R₄ are each independently hydrogen atom, a fatty acid residue or a polycarboxylic acid residue, and R₅ and R₆ are each independently hydrogen atom, a fatty acid residue or a polycarboxylic acid residue, provided that all of R₁ to R₄ do not represent hydrogen atom at the same time, and both of R₅ and R₆ do not represent hydrogen atom at the same time.

The fatty acid for constituting the component (A) may preferably be a straight-chain or branched fatty acid having 5 to 28 carbon atoms. More preferably used are branched fatty acids. Examples of those branched fatty acids are pivalic acid, isoheptanoic acid, 4-ethylpentanoic acid, isooctylic acid, 2-ethylhexanoic acid, 4,5-dimethylhexanoic acid, 4-propylpentanoic acid, isononanoic acid, 2-ethylheptanoic acid, 3,5,5-trimethylhexanoic acid, isodecanoic acid, isododecanoic acid, 2-methyldecanoic acid, 3-methyldecanoic acid, 4-methyldecanoic acid, 5-methyldecanoic acid, 6-methyldecanoic acid, 7-methyldecanoic acid, 9-methyldecanoic acid, 6-ethylnonanoic acid, 5-propyloctanoic acid, isolauric acid, 3-methylhendecanoic acid, 6-propylnonanoic acid, isotridecanoic acid, 2-methyldodecanoic acid, 3-methyldodecanoic acid, 4-methyldodecanoic acid, 5-methyldodecanoic acid, 11-methyldodecanoic acid, 7-propyldecanoic acid, isomyristic acid, 2-methyltridecanoic acid, 12-methyltridecanoic acid, isopalmitic acid, 2-hexyldecanoic acid, 14-methylpentadecanoic acid, 2-ethyltetradecanoic acid, isostearic acid, methyl-branched isostearic acid, 2-heptylundecanoic acid, 2-isoheptylisoundecanoic acid, 2-ethylhexadecanoic acid, 14-ethylhexadecanoic acid, 14-methylheptadecanoic acid, 15-methylheptadecanoic acid, 16-methylheptadecanoic acid, 2-butyltetradecanoic acid, isoarachic acid, 3-methylnonadecanoic acid, 2-ethyloctadecanoic acid, isohexacosanoic acid, 24-methylheptacosanoic acid, 2-ethyltetracosanoic acid, 2-butyldocosanoic acid, 2-hexylicosanoic acid, 2-octyloctadecanoic acid and 2-decylhexadecanoic acid. Those fatty acids can be used alone or in combination. Among those fatty acids, preferred are fatty acids having 8 to 18 carbon atoms, in particular, branched saturated fatty acids having 8 to 18 carbon atoms, such as isooctylic acid (preferably, 2-ethylhexanoic acid and 4,5-dimethylhexanoic acid), isononanoic acid (preferably, 2-ethylheptanoic acid and 3,5,5-trimethylhexanoic acid), isopalmitic acid, isotridecanoic acid, isostearic acid (preferably, methyl-branched isostearic acid, 2-heptylundecanoic acid and 2-isoheptylisoundecanoic acid), and the like. Of those fatty acids, especially preferred are isooctylic acid, more preferably 2-ethylhexanoic acid, and isostearic acid, more preferably methyl-branched isostearic acid.

With respect to the straight-chain fatty acids, there can be employed straight-chain saturated fatty acids having 6 to 28 carbon atoms including saturated fatty acids such as caproic acid, caprylic acid, octylic acid, nonylic acid, decanoic acid, dodecanoic acid, lauric acid, tridecanoic acid, myristic acid, palmitic acid, stearic acid, behenic acid and the like; and straight-chain unsaturated fatty acids such as caproleic acid, undecylenic acid, myristoleic acid, palmitoleic acid, oleic acid, elaidic acid, gondoic acid, erucic acid, brassidic acid and the like. Those fatty acids can be used alone or in combination.

The ester compound represented by formula (I) as the component (A) in the present invention includes one or more monoesters, diesters, triesters and tetraesters. The ester compound represented by formula (II) includes one or more monoesters and diesters. The ester compound represented by formula (I) and formula (II) means a mixture of two or more ester compounds separately selected from the ester compounds of formula (I) and the ester compounds of formula (II).

Further, with respect to the component (A) for use in the present invention, at least one of R₁ to R₄ in the formula (I) may preferably be hydrogen atom. Also, at least one of R₅ or R₆ in the formula (II) may preferably be hydrogen atom.

In the present invention, it is preferable that the ester compound include diesters and triesters each of which has a basic skeleton represented by the above-mentioned formula (I) in an amount of 20 to 94% by mass, more preferably 40 to 80% by mass in total.

Preferably, the component (A) used in the present invention may be a mixture of reaction products of erythritol and/or erythritol condensate with isooctylic acid, represented by formula (I-1) and/or formula (II-1). In this case, it is preferable that the mixture contain the monoester, diester, triester and tetraester, each having a basic skeleton as represented by formula (I-1), in amounts of 0 to 10, 0 to 30, 18 to 70 and 6 to 75% by mass, respectively, more preferably, 0 to 3, 0 to 20, 13 to 70 and 8 to 60% by mass, respectively, and most preferably, 0 to 3, 3 to 20, 30 to 70 and 8 to 40, respectively. Also, it is preferable that the mixture contain the monoester and diester, each having a basic skeleton as represented by formula (II-1), in amounts of 0 to 10 and 0 to 50% by mass respectively, more preferably, 0 to 3 and 0 to 35% by mass, respectively, and most preferably, 0 to 3 and 5 to 35% by mass, respectively. 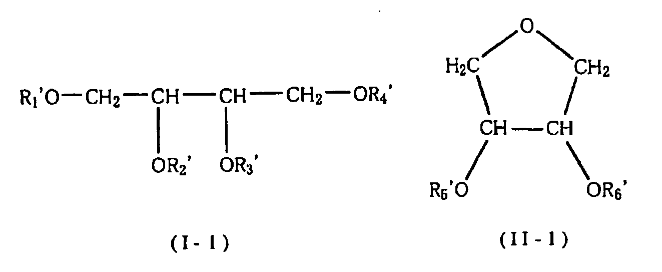
wherein R₁' to R₄' are each independently hydrogen atom or isooctylic acid residue and R₅' and R₆' are each independently hydrogen atom or isooctylic acid residue, provided that all of R₁' to R₄' do not represent hydrogen atom at the same time, and both of R₅' and R₆' do not represent hydrogen atom at the same time.

In the above formulas, the isooctylic acid residue includes -C(=O)-(CH₂CH₃)CH-(CH₂)₃-CH₃ [2-ethylhexanoic acid] and -C(=O)-(CH₂)₂₋(CH₃)CH-(CH₃)CH-CH₃ [4,5-dimethylhexanoic acid].

The amount ratios of the monoester, diester, triester and tetraester previously specified in the case of the formula (I-1) apply to the case of formula (I); and the amount ratios of the monoester and diester previously specified in the case of the formula (II-1) apply to the case of formula (II).

The polycarboxylic acid used in the present invention to prepare the polycondensate as the component (A) in the present invention may preferably include dibasic carboxylic acids having 2 to 10 carbon atoms such as succinic acid, adipic acid, azelaic acid, sebacic acid and the like, and more preferably, dibasic saturated carboxylic acids having 4 to 10 carbon atoms. Especially, succinic acid is preferred. Those polycarboxylic acids can be used alone or in combination.

To prepare the polycondensate as the component (A) in the present invention, it is preferable to use as the raw material a mixture of a branched fatty acid (preferably, a branched saturated fatty acid) having 8 to 18 carbon atoms and a dibasic carboxylic acid having 2 to 10 carbon atoms (preferably, a dibasic carboxylic acid having 4 to 10 carbon atoms); and a mixture of a branched fatty acid (preferably, a branched saturated fatty acid) having 8 to 18 carbon atoms, a straight-chain fatty acid (a straight-chain saturated fatty acid) having 8 to 18 carbon atoms, and a dibasic carboxylic acid having 2 to 10 carbon atoms (preferably, a dibasic carboxylic acid having 4 to 10 carbon atoms). In this case, the branched fatty acid and the dibasic carboxylic acid may preferably be used with a molar ratio of branched fatty acid / dibasic carboxylic acid ranging from 70/30 to 95/5, and the branched fatty acid, the straight-chain fatty acid and the dibasic carboxylic acid may preferably be used with a molar ratio of (branched fatty acid and straight-chain fatty acid) / dibasic carboxylic acid ranging from 70/30 to 95/5.

The component (A) for use in the present invention preferably has a hydroxyl value (hereinafter referred to as "OHV") ranging from 10 to 150, more preferably 20 to 120, and most preferably 30 to 110. The OHV of 35 or more and 110 or less is also preferable. When a mixture of the compounds above specified is used as the component (A), it is preferable that the total OHV of the mixture be within the above-mentioned range. When the OHV is within the above-mentioned range, the hydration tendency is improved to easily offer a moisturizing feel. The term OHV herein used is a value determined by the hydroxyl value measurement test method in accordance with the Japanese Standards of Cosmetic Ingredients. Preferably, the component (A) for use in the present invention may assume a liquid state at room temperature, preferably having a viscosity of 30 to 30,000 mPa·s, more preferably 50 to 30,000 mPa·s, and most preferably 100 to 30,000 mPa·s when measured with a Brookfield viscometer at 25°C. The viscosity of 50 mPa·s or more is preferable, and the viscosity of 10,000 mPa·s or less is preferable, more preferably 5,000 mPa·s or less, and further preferably 1,100 mPa·s or less.

The component (A) for use in the present invention can be prepared, for example, by adding 1.5 to 3.5 equivalents of a fatty acid and/or polycarboxylic acid to one equivalent of erythritol, and carrying out a reaction of esterification and/or dehydration condensation at 180 to 240°C for 6 to 40 hours in the absence or presence of a catalyst (e.g., tin chloride). After completion of the reaction, the catalyst is removed from the reaction mixture by adsorption treatment or the like, and low-molecular weight components including an unreacted raw material are eliminated by distillation or the like, thereby obtaining a final product.

The content of the component (A) in the oil-in-water emulsion type cosmetic preparation of the present invention is not particularly limited, but may preferably be in the range of 0.1 to 30% by mass, more preferably 1 to 10% by mass, with respect to the total mass of the oil-in-water emulsion type cosmetic preparation. When the content of the component (A) is within the above-mentioned range, it becomes possible to obtain excellent products in terms of the long-lasting moisturizing feel and feeling realized by the use of it.

In the present invention, it is preferable that the component (A) include the ester compound of formula (I) and/or formula (II) and/or the polycondensate of erythritol, fatty acid and polycarboxylic acid in an amount of 2% by mass or more and 30% by mass or less, more preferably 8% by mass or less, and further preferably 5% by mass or less.

As the component (B) in the oil-in-water emulsion type cosmetic preparation of the present invention, any nonionic surfactants with an HLB of 10 or more and any ionic surfactants with an HLB of 10 or more that are conventionally used for the cosmetics are usable with no limitation. Specific examples of the nonionic surfactant with an HLB of 10 or more are polyglycerol fatty acid esters, polyoxyethylene hardened castor oil derivatives, polyoxyethylene castor oil derivatives; polyoxyethylene sorbitan fatty acid esters such as polyoxyethylene sorbitan monostearate, polyoxyethylene sorbitan monooleate, polyoxyethylene sorbitan trioleate and the like, polyoxyethylene glycerol fatty acid esters such as polyoxyethylene glycerol monooleate, polyoxyethylene trioleate, polyoxyethylene monoisostearate and the like; polyethylene glycol fatty acid esters such as polyethylene glycol monoisostearate and the like, polyoxyethylene addition type nonionic surfactants such as polyoxyethylene alkyl ether, polyoxypropylene alkyl ether, polyoxyethylene alkylphenyl ether, polyoxyethylene phytostanol ether, polyoxyethylene phytosterol ether, polyoxyethylene cholestanol ether, polyoxyethylene cholesteryl ether, polyoxyalkylene-modified organopolysiloxane and the like. Of those nonionic surfactants, preferably used are those with an HLB of 10 to 15, in particular, polyoxyethylene sorbitan fatty acid esters, more preferably polyoxyethylene sorbitan monostearate, polyoxyethylene sorbitan monooleate and polyoxyethylene sorbitan trioleate.

The ionic surfactant with an HLB of 10 or more includes anionic surfactants, amphoteric surfactants and cationic surfactants. Examples of the anionic surfactants are fatty acids such as stearic acid, lauric acid and the like, and soaps thereof, alkylphosphates, polyoxyethylene alkyl ether phosphates, alkyl sulfates, polyoxyethylene alkyl ether sulfates and the like. When the fatty acid soap is used as the anionic surfactant, sodium salt or potassium salt of fatty acid prepared in advance may be used. Alternatively, an alkali such as sodium hydroxide, potassium hydroxide or the like may be mixed with a fatty acid in the preparation of the cosmetic formulation. The amphoteric surfactants include acetate betaine, alkylaminoacetate betaine, imidazolinium betaine, and the like. The cationic surfactants include alkylammonium salts, alkylbenzyl ammonium salts and the like. The nonionic surfactants with an HLB of less than 10 including sorbitan tristearate, sorbitan monooleate, polyoxyethylene sorbitan sesquioleate and the like can be used to such an extent that the effects of the present invention will not be impaired when contained as an emulsifying aid or a dispersant for a powder if added.

The content of the component (B) in the oil-in-water emulsion type cosmetic preparation of the present invention is not particularly limited, but may preferably be in the range of 0.01 to 10% by mass, more preferably 0.1 to 2% by mass, with respect to the total mass of the oil-in-water emulsion type cosmetic preparation. When the content of the component (B) is within the above-mentioned range, there can be obtained the cosmetic preparations that can smoothly spread to ensure the feeling realized by the use of it and exhibit excellent emulsion stability over an extended period of time.

The water-base ingredient used as the component (C) in the oil-in-water emulsion type cosmetic preparation of the present invention includes water and water-soluble substances that can serve as solvents. For example, there can be employed lower alcohols such as ethyl alcohol, butyl alcohol and the like; glycols such as propylene glycol, 1,3-butylene glycol, dipropylene glycol, polyethylene glycol and the like; glycerols such as glycerin, diglycerin, polyglycerin and the like; and aqueous extracts from plants such as aloe vera, witch hazel, hamamelis, cucumber, lemon, lavender, rose and the like. One or more water-base ingredients may be selected from the above-mentioned group. It is preferable to use one or more water-base ingredients selected from the group consisting of, water, ethyl alcohol, 1,3-butylene glycol, dipropylene glycol and glycerin.

The content of the component (C) in the oil-in-water emulsion type cosmetic preparation of the present invention is not particularly limited, but may preferably be in the range of 40 to 95% by mass, more preferably 70 to 90% by mass, with respect to the total mass of the oil-in-water emulsion type cosmetic preparation. When the content of the component (C) is within the above-mentioned range, excellent products can be obtained in terms of the feeling realized by the use of it, i.e., ease of spreading to skin, and the non-sticky feel. In addition, it is preferable that the component (C) contain water in an amount of 50% by mass or more.

In addition to the above-mentioned essential ingredients, various ingredients conventionally incorporated into the formulation for the cosmetic preparations, for example, an oil ingredient other than the component (A), a powder material, a water-soluble polymer, a moisturizing agent, a UV absorber, an antioxidant, a preservative, enzymes including lipase, protease and the like, a variety of pharmaceutical drugs including resorcin, sulfur and the like, a refrigerant, a coloring agent, a perfume and the like may be blended into the oil-in-water emulsion type cosmetic preparation of the present invention so far as the effects of the present invention will not be damaged.

Regardless of origin of oil, i.e., whether the oil ingredient is from animal oil, vegetable oil, or synthetic oil that is typically used for cosmetics, and regardless of properties of oil, i.e., whether the oil ingredient is a solid oil, semi-solid oil, liquid oil, volatile oil or the like, the oil ingredient other than the component (A) may include hydrocarbons, fats and oils, waxes, hardened oils, ester oils, fatty acids, higher alcohols, silicone oils, fluorinated oils, oil gelling agents and the like. More specifically, there can be employed the hydrocarbons such as liquid paraffin, squalane, vaseline, polyisobutylene, polybutene, montan wax, polyethylene wax, carnauba wax, microcrystalline wax, Fischer-Tropsch wax, paraffin wax and the like; amino acid ester oils such as di(cholesteryl/behenyl/octyldodecyl) N-lauroyl-L-glutamate, di(phytosteryl/behenyl/octyldodecyl) N-lauroyl-L -glutamate and the like, polyhydric alcohol fatty acid esters such as butyrospermum parkii (shea butter); hexaglycerin fatty acid ester, decaglycerin fatty acid ester, glyceryl oligoester (adipate/2-ethylhexanoate/stearate), dipentaerythrityl 12-hydroxystearate, cholesterol derivatives and phytosterol derivatives, such as cholesterol, cholestanol, dehydrocholesterol, cholesteryl lanolate, cholesteryl isostearate, cholesteryl 12-hydroxystearate, cholesteryl ricinoleate, cholesteryl macadamiate and the like; lanolin derivatives and polyoxyalkylene-modified oils thereof, such as lanolin, super-refined lanolin, liquid lanolin, reduced lanolin, liquid lanolin acetate, lanolin alcohol, hydrogenated lanolin alcohol, lanolin fatty acid and the like; fats and oils, such as Japan wax, olive oil, castor oil, mink oil, macadamia nut oil and the like; waxes such as beeswax, spermaceti, carnauba wax, candelilla wax and the like; esters such as jojoba oil, isopropyl myristate, isopropyl palmitate, octyldodecyl myristate, glyceryl trioctanoate (glyceryl tri(2-ethylhexanoate)), polyglyceryl diisostearate, diisostearyl malate, pentaerythrityl rosin, neopentyl glycol dioctanoate, neopentyl glycol dicaprate and the like; fatty acids such as stearic acid, lauric acid, myristic acid, behenic acid, isostearic acid, oleic acid and the like; higher alcohols such as stearyl alcohol, cetyl alcohol, lauryl alcohol, oleyl alcohol, isostearyl alcohol, behenyl alcohol and the like; silicones such as methyl polysiloxane, methylphenyl polysiloxane, decamethylcyclopentasiloxane, octamethylcyclotetrasiloxane, trimethylsiloxy silicate, methyl polysiloxane with high degree of polymerization, methylphenyl polysiloxane with high degree of polymerization, dimethyl polysiloxane with high degree of polymerization, alkoxy-modified polysiloxane and the like; fluorinated oils such as perfluorodecane, perfluorooctane, perfluoropolyether and the like; and oil gelling agents such as dextrin fatty acid esters, sucrose fatty acid esters, starch fatty acid esters, potassium stearate, 12-hydroxystearic acid and the like. Those may be used alone or in combination.

The powder may include any powder materials generally used for cosmetics. Inorganic powders, optical powders, organic powders, pigment powders, metallic powders, composite powders and the like can be used without any limitation on the shape thereof, i.e., whether they may be spheres, plates, needles or the like, the particle diameter thereof, i.e., whether they may be aerosol particles, fine particles, pigment-grade particles or the like, and the particle structure thereof, i.e., whether they may be porous, non-porous or the like. Specific examples of the powder materials are inorganic white pigments such as titanium oxide, silicone-treated titanium oxide, zinc oxide, cerium oxide, barium sulfate and the like; inorganic colored pigments such as iron oxide, iron oxide black, iron oxide red, iron oxide yellow, silicone-treated iron red, carbon black, sintered material of titanium and titanium oxide, chromium oxide, chromium hydroxide, iron blue, ultramarine and the like; white extender pigments such as talc, silicone-treated talc, muscovite, phlogopite, lepidolite, biotite, synthetic mica, sericite, synthetic sericite, kaolin, silicon carbide, bentonite, smectite, silica, aluminum oxide, magnesium oxide, zirconium oxide, antimony oxide, diatomite, aluminum silicate, aluminum magnesium metasilicate, calcium silicate, barium silicate, magnesium silicate, calcium carbonate, magnesium carbonate, hydroxyapatite, boron nitride and the like; optical powders such as titanium dioxide-coated mica, titanium dioxide-coated bismuth oxychloride, iron oxide-coated titanated mica, iron blue-coated titanated mica, carmine-treated titanated mica, silicone-treated titanated mica, bismuth oxychloride, fish scale flakes, laminated powder of epoxy resin coated polyethylene terephthalate · aluminum, laminated film powder of polyethylene terephthalate · polyolefin, spherical PMMA powder, laminated film powder of polyethylene terephthalate · polymethyl methacrylate and the like; organic high-molecular weight resin powders such as polyamide resin, polyethylene resin, polyacrylic resin, polyester resin, fluoroplastic, cellulose resin, polystyrene resin, copolymer resin including styrene - acryl copolymer resin, polypropylene resin, silicone resin, urethane resin and the like; organic low-molecular weight powders such as zinc stearate, N-acyl-lysine and the like; organic natural powders such as starch, silk powder, cellulose powder and the like; organic pigment powders such as Red No. 201, Red No. 202, Red No. 205, Red No. 226, Red No. 228, Orange No. 203, Orange No. 204, Blue No. 404, Yellow No. 401 and the like; organic pigment powders of zirconium, barium or aluminum lake, such as Red No. 3, Red No. 104, Red No. 106, Orange No. 205, Yellow No. 4, Yellow No. 5, Green No. 3, Blue No. 1 and the like; metallic powders such as mica, aluminum powder, gold powder, silver powder and the like; composite powders such as titanium oxide fine particles-coated titanated mica, zinc oxide fine particles-coated titanated mica, barium sulfate-coated titanated mica, titanium oxide-containing silicon dioxide, zinc oxide-containing silicon dioxide and the like. Those powder materials may be used alone or in combination, and another composite powders made from the above powders can also be used.

The UV absorber includes, for example, benzophenone compounds, PABA compounds, cinnamates, salicylates, 4-tert-butyl-4'-methoxydibenzoylmethane, oxybenzone and the like.

Examples of the water-soluble polymer include natural polymers such as xanthan gum, guar gum, sodium chondroitin sulfate, sodium hyaluronate, gum arabic, sodium alginate, carrageenan, water-swelling clay minerals and the like; semi-synthetic polymers such as methyl cellulose, hydroxyethyl cellulose, carboxymethyl cellulose and the like; and synthetic polymers such as carboxyvinyl polymer, alkyl-added carboxyvinyl polymer, polyvinyl alcohol, polyvinylpyrrolidone, sodium polyacrylate and the like. Examples of the moisturizing agent include protein, mucopolysaccharide, collagen, elastin, keratin, triethanolamine and the like.

The antioxidant includes, for example, tocopherols, ascorbic acid, magnesium L-ascorbate phosphate and the like. The beauty ingredient includes, for example, vitamins, anti-inflammatory agents, crude drugs and the like, and the preservative includes, for example, p-hydroxybenzoate, paraben, phenoxyethanol and the like.

Particularly preferable formulation examples according to the present invention will be shown below.

### Formulation Example 1

An oil-in-water emulsion type cosmetic preparation comprising:
Component (A): an ester compound of formula (I) and/or formula (II), a polycondensate of erythritol and/or erythritol condensate, fatty acid and polycarboxylic acid, or a mixture thereof, which has an OHV of 35 to 110;
Component (B): a polyoxyethylene sorbitan fatty acid ester with an HLB of 10 to 15; and
Component (C): one or more water-base ingredients selected from the group consisting of water, ethyl alcohol, 1,3-butylene glycol, dipropylene glycol and glycerin.

### Formulation Example 2

An oil-in-water emulsion type cosmetic preparation comprising:
Component (A): an ester compound of erythritol and 2-ethylhexanoic acid, an ester compound of erythritol and isostearic acid, a polycondensate of erythritol, isostearic acid and succinic acid, or a mixture thereof;
Component (B): polyoxyethylene sorbitan monostearate, polyoxyethylene sorbitan monooleate, or polyoxyethylene sorbitan trioleate; and
Component (C): one ore more water-base ingredients selected from the group consisting of water, ethyl alcohol, 1,3-butylene glycol, dipropylene glycol and glycerin.

The process for manufacturing the oil-in-water emulsion type cosmetic preparation of the present invention is not particularly limited. For example, the cosmetic preparation can be obtained in such a manner that the component (A), and other oily ingredients if necessary, are heated and added to a mixture of the component (C) and other ingredients which is also heated, and both are emulsified to form a mixture in the presence of the component (B), and then cooled.

The oil-in-water emulsion type cosmetic preparation of the present invention may be made into any product form, for example, basic skin care preparations for face, hands, feet and body, such as lotion, sunscreen, emulsion, cream, essence, cosmetic liquid for a non-woven mask which is impregnated therewith, face pack, hairdressing, hair tonic and the like; and make-up preparations such as lipstick, liquid rouge, gloss, foundation, eye-shadow, base cream and the like.

The present invention will now be explained in detail by referring to the following examples, which are not intended to be limiting of the present invention.

### Examples

### Preparation Example 1

### Preparation of ester compound of erythritol and 2-ethylhexanoic acid

A four-necked flask (300 mL) equipped with a stirrer, a thermometer, a nitrogen gas inlet, and a reflux condenser was charged with 178 g (1.24 mol) of 2-ethylhexanoic acid (octylic acid made by Kyowa Hakko Kogyo Co., Ltd.) and 72 g (0.59 mol) of erythritol (erythritol made by Nikken Chemicals Co., Ltd.). Xylene was added as a solvent for reflux in an amount of 5% by mass of the total mass of the charged materials. The mixture was allowed to react at 180 to 240°C for 20 hours with stirring. After completion of the reaction, xylene serving as the solvent for reflux was distilled away under reduced pressure, the decolorization treatment was carried out using activated clay and deodorization and distillation were performed by the conventional methods, so that 142 g of a desired ester compound of erythritol and 2-ethylhexanoic acid, having a hydroxyl value of 101 was obtained. The viscosity was 51 mPa·s when measured with a Brookfield viscometer (25°C).

The contents of diester, triester and tetraester having the basic skeleton of formula (I-1), and the content of diester having the basic skeleton of formula (II-1) were found to be 7.7, 41.5, 20.4 and 28.9% by mass, respectively.

### Preparation Example 2

### Preparation of ester compound of erythritol and isostearic acid

A four-necked flask (300 mL) equipped with a stirrer, a thermometer, a nitrogen gas inlet, and a reflux condenser was charged with 222 g (0.78 mol) of isostearic acid ("Prisorin ISAC3505" made by Uniquema) and 37 g (0.30 mol) of erythritol (erythritol made by Nikken Chemicals Co., Ltd.). Xylene was added as a solvent for reflux in an amount of 5% by mass of the total mass of the charged materials. The mixture was allowed to react at 180 to 240°C for 13 hours with stirring. After completion of the reaction, xylene serving as the solvent for reflux was distilled away under reduced pressure, the decolorization treatment was carried out using activated clay and deodorization and distillation were performed by the conventional methods, so that 204 g of a desired ester compound of erythritol and isostearic acid, having a hydroxyl value of 50 was obtained. The viscosity was 336 mPa·s when measured with a Brookfield viscometer (25°C).

### Preparation Example 3

### Preparation of polycondensate of erythritol, isostearic acid and succinic acid

A four-necked flask (300 mL) equipped with a stirrer, a thermometer, a nitrogen gas inlet, and a reflux condenser was charged with 185 g (0.65 mol) of isostearic acid ("Prisorin ISAC3505" made by Uniquema) and 37 g (0.30 mol) of erythritol (erythritol made by Nikken Chemicals Co., Ltd.). Xylene was added as a solvent for reflux in an amount of 5% by mass of the total mass of the charged materials. The mixture was allowed to react at 180 to 210°C for 10 hours with stirring and cooled. To the reaction mixture, 16 g (0.16 mol) of succinic anhydride ("Rikacid SA" made by New Japan Chemical Co., Ltd.) was added, and the reaction was carried out again at 120 to 230°C for 16 hours with stirring. After completion of the reaction, xylene serving as the solvent for reflux was distilled away under reduced pressure, the decolorization treatment was carried out using activated clay and deodorization and distillation were performed by the conventional methods, so that 143 g of a desired polycondensate of erythritol, isostearic acid and succinic acid, having a hydroxyl value of 39 was obtained. The viscosity was 1130 mPa·s when measured with a Brookfield viscometer (25°C).

### Examples 1 to 10 and Comparative Examples 1 to 3 (Essence solution)

Essences with the formulations as shown in the following Table 1 were produced by the method shown below. The skin feel (1) (a non-sticky feel and a long-lasting moisturizing feel), and the long-term emulsion stability (2) of each essence were evaluated in such a manner as described later. The results are also shown in Table 1.

**Table 1 (unit: % by mass)**

| No. | Ingredients | Examples | | | | | | | | | |
|---|---|---|---|---|---|---|---|---|---|---|---|
| | | 1 | 2 | 3 | 4 | 5 | 6 | 7 | 8 | 9 | 10 |
| 1 | Polyoxyethylene sorbitan monooleate (20E.O.) (HLB:15) | 0.5 | 0.5 | 0.01 | 10 | 0.5 | 0.5 | 0.5 | 0.5 | 2 | 0.5 |
| 2 | Sorbitan sesquioleate (HLB:3.7) | 0.1 | 0.1 | 0.01 | 0.1 | 0.1 | 0.1 | - | 0.1 | 0.1 | 0.1 |
| 3 | Ester compound of Prep. Exam. 1 | 2 | - | - | - | - | - | - | - | - | - |
| 4 | Ester compound of Prep. Exam. 2 | - | 2 | 2 | 2 | 0.1 | 30 | 2 | 1 | 8 | 2 |
| 5 | Polycondensate of Prep. Exam. 3 | - | - | - | - | - | - | - | 1 | - | - |
| 6 | Glyceryl trioctanoate | - | - | - | - | - | - | - | - | - | - |
| 7 | Cetyl 2-ethylhexanoate | 5 | 5 | 5 | 5 | 5 | 5 | 5 | 5 | 5 | 5 |
| 7 | Ethyl alcohol | 10 | 10 | 10 | 10 | 10 | 10 | 10 | 10 | 10 | 10 |
| 8 | Perfume | 0.1 | 0.1 | 0.1 | 0.1 | 0.1 | 0.1 | 0.1 | 0.1 | 0.1 | 0.1 |
| 9 | Dipropylene glycol | 10 | 10 | 10 | 10 | 10 | 10 | 10 | - | 10 | 20 |
| 10 | Glycerin | 10 | 10 | 10 | 10 | 10 | 10 | 10 | 20 | 10 | - |
| 11 | Preservative | 0.1 | 0.1 | 0.1 | 0.1 | 0.1 | 0.1 | 0.1 | 0.1 | 0.1 | 0.1 |
| 12 | Purified water | 62.2 | 62.2 | 62.78 | 52.7 | 64.1 | 34.2 | 62.3 | 62.2 | 54.7 | 62.2 |
| Total | | 100 | 100 | 100 | 100 | 100 | 100 | 100 | 100 | 100 | 100 |
| Evaluation items & Results | | | | | | | | | | | |
| Non-sticky skin feel | | A | A | A | B | A | B | A | B | B | B |
| Long-lasting moisturizing feel | | A | A | A | A | B | A | A | A | A | A |
| Long-term emulsion stability | | A | A | B | A | A | B | B | A | A | A |

| No. | Ingredients | Comparative Examples | | |
|---|---|---|---|---|
| | | 1 | 2 | 3 |
| 1 | Polyoxyethylene sorbitan monooleate (20E.O.) (HLB:15) | - | 0.5 | 0.5 |
| 2 | Sorbitan sesquioleate (HLB:3.7) | 0.1 | 0.1 | 0.1 |
| 3 | Ester compound of Prep.Exam. 1 | - | - | - |
| 4 | Ester compound of Prep. Exam. 2 | 2 | - | - |
| 5 | Polycondensate of Prep. Exam. 3 | - | - | - |
| 6 | Glyceryl trioctanoate | - | - | 2 |
| 7 | Cetyl2-ethylhexanoate | 5 | 5 | 5 |
| 8 | Ethyl alcohol | 10 | 10 | 10 |
| 9 | Perfume | 0.1 | 0.1 | 0.1 |
| 10 | Dipropylene glycol | 10 | 10 | 10 |
| 11 | Glycerin | 10 | 10 | 10 |
| 12 | Preservative | 0.1 | 0.1 | 0.1 |
| 13 | Purified water | 62.7 | 64.2 | 62.2 |
| Total | | 100 | 100 | 100 |
| Evaluation items & Results | | | | |
| Non-sticky skin feel | | C | C | C |
| Long-lasting moisturizing feel | | C | D | C |
| Long-term emulsion stability | | D | B | D |

### (Manufacturing process)

Step A: The ingredients 1 through 7 were fused and mixed at 80°C.
Step B: The ingredients 8 through 13 were heated to 80°C and added to the mixture obtained in the step A, and the resultant mixture was subjected to emulsification.
Step C: The emulsified product obtained in the step B was cooled to obtain an essence.

### (Evaluation method for skin feel)

To evaluate a non-sticky feel and a long-lasting moisturizing feel, a sensory evaluation test was conducted using 30 female panelists who had worn make-up for 10 years or more. Each panelist assessed each sample product on the following absolute evaluation scale (a) (a 0-to-6 scale). From the average score the product was rated on four levels according to the evaluation criteria (b) shown below. The long-lasting moisturizing feel was assessed in such a manner that each sample was applied to the lips and the moisturizing effect was evaluated after a lapse of 3 hours of daily routine.

### (a) Absolute evaluation scale

| <Score> : | <Evaluation> |
|---|---|
| 6: | Excellent |
| 5: | Good |
| 4: | Fair |
| 3: | Ordinary |
| 2: | Slightly poor |
| 1: | Poor |
| 0: | Very poor |

### (b) Four-level rating criteria

| <Average score>: | <Rating> |
|---|---|
| More than 5: | Excellent (A) |
| More than 3 and 5 or less: | Good (B) |
| More than 1 and 3 or less: | Slightly poor (C) |
| 1 or less: | Poor (D) |

### (Evaluation method for long-term emulsion stability)

Each sample was allowed to stand in a thermostat of 50°C for one month. Then, the sample was rated according to the following four-level rating criteria (c) based on the change of appearance (separation and creaming).

### (c) Four-level rating criteria

| <Degree of change> : | <Rating> |
|---|---|
| No change: | A |
| Slightly changed: | B |
| Substantially changed: | C |
| Considerably changed: | D |

As is apparent from the results shown in Table 1, the essences of the present invention obtained in Examples 1 to 10 were superior to those of Comparative Examples 1 to 3 in terms of the non-sticky skin feel, the long-lasting moisturizing feel, and the long-term emulsion stability. In contrast to the essences of the present invention, the essence of Comparative Example 2 not using the component (A) that is an element for constituting the present invention was unsatisfactory especially in terms of the long-lasting moisturizing effect. The essence of Comparative Example 1 not using the component (B) that is another element for constituting the present invention was unsatisfactory especially in terms of the long-term stability. The essence of Comparative Example 3 using glyceryl trioctanoate instead of the component (A) that is the element for constituting the present invention was unsatisfactory especially in terms of the long-term emulsion stability.

### Example 11: Emulsion

| (Ingredients) | (% by mass) |
|---|---|
| 1. Sorbitan monooleate (HLB: 4.3) | 0.1 |
| 2. Polyoxyethylene sorbitan monostearate (20E.O.) (HLB: 14.9) | 2.0 |
| 3. Ester compound of Preparation Example 2 | 10.0 |
| 4. Perfume | 0.02 |
| 5. Glyceryl trioctanoate | 1.0 |
| 6. Dipropylene glycol | 5.0 |
| 7. Glycerin | 5.0 |
| 8. Sodium alginate | 0.5 |
| 9. Carboxyvinyl polymer | 0.2 |
| 10. Preservative (Ethyl p-hydroxybenzoate) | 0.1 |
| 11. Purified water | 75.98 |
| 12. Magnesium L- ascorbate phosphate | 0.1 |
| Total | 100 |

### (Manufacturing process)

Step A: The ingredients 1 through 5 were fused and mixed at 80°C.
Step B: The ingredients 6 through 12 were heated to 80°C and added to the mixture obtained in the step A. The resultant mixture was subjected to emulsification.
Step C: The emulsified product obtained in the step B was cooled to produce an emulsion.

The emulsion of Example 11 was excellent in terms of the non-sticky skin feel and the long-lasting moisturizing feel. Also, the emulsion showed excellent stability without separation and creaming over a long period of time.

### Example 12: Eye cream

| (Ingredients) | (% by mass) |
|---|---|
| 1. Sorbitan tristearate (HLB: 2.1) | 0.05 |
| 2. Polyoxyethylene sorbitan trioleate (20E.O.) (HLB:11.0) | 0.1 |
| 3. Di(phytosteryl/behenyl/octyldodecyl) N-lauroyl-L-glutamate | 0.5 |
| 4. Microcrystalline wax | 0.5 |
| 5. Polybutene | 1.5 |
| 6. Stearyl alcohol | 2.5 |
| 7. Ester compound of Preparation Example 1 | 1.0 |
| 8. Dimethyl polysiloxane | 0.5 |
| 9. Dipropylene glycol | 5.0 |
| 10. Glycerin | 5.0 |
| 11. Sodium alginate | 0.1 |
| 12. Preservative (Ethyl p-hydroxybenzoate) | 0.1 |
| 13. Purified water | 83.13 |
| 14. Perfume | 0.02 |
| Total | 100 |

### (Manufacturing process)

Step A: The ingredients 1 through 8 were fused and mixed at 80°C.
Step B: The ingredients 9 through 14 were heated to 80°C and added to the mixture obtained in the step A. The resultant mixture was subjected to emulsification.
Step C: The emulsified product obtained in the step B was cooled to produce an eye cream.
   The eye cream of Example 12 was excellent in terms of the non-sticky skin feel and the long-lasting moisturizing feel. Also, excellent emulsion stability was obtained without separation and creaming over a long period of time.

### Example 13: Foundation

| (Ingredients) | (% by mass) |
|---|---|
| 1. Decamethylcyclopentasiloxane | 5.0 |
| 2. Organic silicone resin solution ^{Note (1)} | 5.0 |
| 3. Polycondensate of Preparation Example 2 | 1.0 |
| 4. Neopentyl glycol dicaprate | 2.0 |
| 5. Stearic acid | 1.5 |
| 6. Sorbitan tristearate (HLB: 2.1) | 0.05 |
| 7. Polyoxyethylene sorbitan trioleate (20E.O.) (HLB:11.0) | 0.2 |
| 8. Triethanolamine | 0.5 |
| 9. Paraben | 0.3 |
| 10. Potassium hydroxide | 0.2 |
| 11. 1,3-butylene glycol | 10.0 |
| 12. Purified water | 53.3 |
| 13. Water-swelling clay mineral ^{Note(2)} | 0.8 |
| 14. Carboxymethyl cellulose | 0.1 |
| 15. Xanthan gum | 0.05 |
| 16. Talc | 5.0 |
| 17. Mica | 3.0 |
| 18. Iron oxide black | 0.3 |
| 19. Iron oxide red | 0.7 |
| 20. Iron oxide yellow | 1.0 |
| 21. Titanium oxide | 8.0 |
| 22. Titanium oxide fine particles | 2.0 |
| Total | 100 |

| | |
|---|---|
| Note (1): "KF-9021" made by Shin-Etsu Chemical Co., Ltd. | |
| Note (2): "Kunipia G-4" made by Kunimine Industries Co., Ltd. | |

### (Manufacturing process)

Step A: The ingredients 1 through 7 were fused and mixed at 80°C.
Step B: The ingredients 8 through 15 were heated to 80°C, where the ingredients 16 through 22 were dispersed using a homomixer.
Step C: The dispersion system obtained in the step B was added to the mixture obtained in the step A, and the resulting mixture was subjected to emulsification and then cooled, thereby obtaining a foundation.

The foundation of Example 13 was excellent in terms of the non-sticky skin feel and the long-lasting moisturizing feel. Also, the preservation stability was excellent without separation and creaming.

### Example 14: Eye-shadow

| (Ingredients) | (% by mass) |
|---|---|
| 1. Sorbitan tristearate (HLB: 2.1) | 0.05 |
| 2. Polyoxyethylene sorbitan trioleate | |
| (20E.O.) (HLB:11.0) | 0.1 |
| 3. Polycondensate of Preparation Example 3 | 5.0 |
| 4. Diisostearyl malate | 2.0 |
| 5. Stearic acid | 0.4 |
| 6. Isostearic acid | 0.5 |
| 7. Purified water | 67.15 |
| 8. Triethanolamine | 0.8 |
| 9. Polyethylene glycol (Molecular weight: 400) | 3.0 |
| 10. Ethanol | 5.0 |
| 11. Paraben | 0.5 |
| 12. Glycerin | 3.0 |
| 13. Red No. 226 | 1.0 |
| 14. Silicone-treated iron red ^{Note (3)} | 1.0 |
| 15. Silicone-treated titanium oxide ^{Note (3)} | 0.5 |
| 16. Silicone-treated titanated mica ^{Note (3)} | 5.0 |
| 17. Silicone-treated talc ^{Note (3)} | 3.0 |
| 18 Spherical PMMA powder (Average particle diameter: 10 µm) | 2.0 |
| Total | 100 |

| | |
|---|---|
| Note (3): treated with dimethyl polysiloxane (5% by mass) | |

Step A: The ingredients 1 through 6 were fused and mixed at 80°C.
Step B: The ingredients 7 through 12 were heated to 80°C, where the ingredients 13 through 18 were added and dispersed using a homomixer.
Step C: The dispersion system obtained in the step B was added to the mixture obtained in the step A, and the resulting mixture was subjected to emulsification and then cooled, thereby obtaining an eye-shadow.

The eye-shadow of Example 14 was excellent in terms of the non-sticky skin feel and the long-lasting moisturizing feel. Also, the emulsion stability was excellent without separation and creaming over a long period of time.

As previously explained in detail, the oil-in-water emulsion type cosmetic preparation of the present invention is provided with excellent properties, which can offer excellent skin feel (non-sticky skin feel and long-lasting moisturizing feel) and exhibit excellent long-term emulsion stability.

## Claims

1. An oil-in-water emulsion type cosmetic preparation comprising:
(A) one or more members selected from the group consisting of ester compounds represented by formula (I) and/or formula (II) which are reaction products of erythritol and/or erythritol condensate with a fatty acid(s); polycondensates of erythritol and/or erythritol condensate, the above-mentioned ester compound(s) and a polycarboxylic acid(s); polycondensates of a fatty acid(s) with a polycondensate(s) of erythritol and/or erythritol condensate with a polycarboxylic acid(s);, and polycondensates of erythritol and/or erythritol condensate, a fatty acid(s) and a polycarboxylic acid(s), 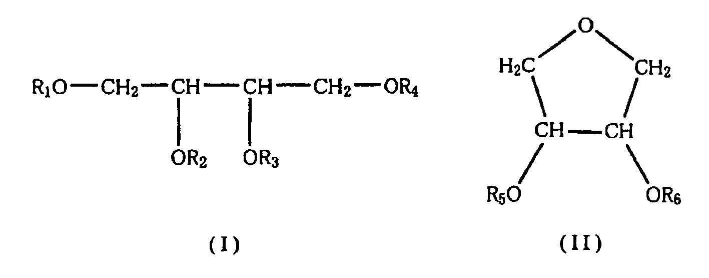
wherein R₁ to R₄ are each independently hydrogen atom, a fatty acid residue or a polycarboxylic acid residue, and R₅ and R₆ are each independently hydrogen atom, a fatty acid residue or a polycarboxylic acid residue, provided that all of R₁ to R₄ do not represent hydrogen atom at the same time, and both of R₅ and R₆ do not represent hydrogen atom at the same time;
(B) a nonionic surfactant with an HLB of 10 or more and/or an ionic surfactant with an HLB of 10 or more; and
(C) a water-base ingredient.

2. The oil-in-water emulsion type cosmetic preparation of claim 1, wherein the fatty acid residue in the formulas (I) and (II) representing component (A) is derived from a straight-chain or branched fatty acid having 5 to 28 carbon atoms.

3. The oil-in-water emulsion type cosmetic preparation of claim 2, wherein the fatty acid residue in the formulas (I) and (II) representing component (A) is derived from the branched fatty acid.

4. The oil-in-water emulsion type cosmetic preparation of claim 3, wherein the fatty acid residue in the formulas (I) and (II) representing component (A) is derived from the branched saturated fatty acid having 8 to 18 carbon atoms.

5. The oil-in-water emulsion type cosmetic preparation of claim 1, wherein the polycarboxylic acid is a dibasic carboxylic acid having 2 to 10 carbon atoms.

6. The oil-in-water emulsion type cosmetic preparation of any one of claims 1 to 5, wherein at least one of R₁ to R₄ in the formula (I) is hydrogen atom.

7. The oil-in-water emulsion type cosmetic preparation of any one of claims 1 to 6, wherein at least one of R₅ or R₆ in the formula (II) is hydrogen atom.

8. The oil-in-water emulsion type cosmetic preparation of claim 1, wherein component (A) is (a) the ester compound of formula (I) and/or formula (II), or (b) the polycondensate of erythritol and/or erythritol condensate, fatty acid, and polycarboxylic acid.

9. The oil-in-water emulsion type cosmetic preparation of claim 1, wherein component (A) is a mixture of (a) the ester compound of formula (I) and/or formula (II) and (b) the polycondensate of erythritol and/or erythritol condensate, fatty acid, and polycarboxylic acid.

10. The oil-in-water emulsion type cosmetic preparation of claim 1, wherein component (A) comprises a diester and a trimester, each having a basic skeleton of formula (I), in an amount of 20 to 94% by mass in total with respect to the total mass of the ester compound.

11. The oil-in-water emulsion type cosmetic preparation of claim 1, wherein component (A) is a mixture of an ester compound of formula (1-1) and/or an ester compound of formula (II-1): 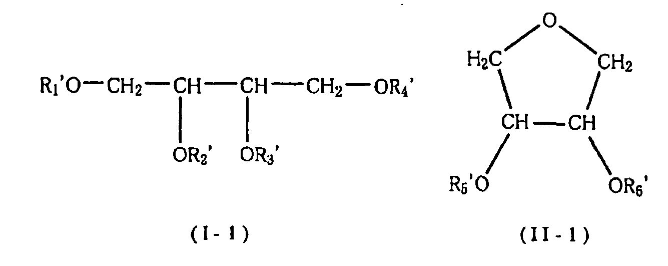
wherein R₁' to R₄' are each independently hydrogen atom or isooctylic acid residue, and R₅' and R₆' are each independently hydrogen atom or isooctylic acid residue, provided that all of R₁' to R₄' do not represent hydrogen atom at the same time, and both of R₅' and R₆' do not represent hydrogen atom at the same time.

12. The oil-in-water emulsion type cosmetic preparation of claim 1, wherein component (A) is obtainable by subjecting one equivalent of the erythritol and 1.5 to 3.5 equivalents of the fatty acid and/or the polycarboxylic acid to esterification and/or dehydration condensation.

13. The oil-in-water emulsion type cosmetic preparation of any one of claims 1 to 12, wherein component (A) has a hydroxyl value (OHV) of 10 to 150.

14. The oil-in-water emulsion type cosmetic preparation of claim 13, wherein the hydroxyl value (OHV) of component (A) is in the range of 20 to 120.

15. The oil-in-water emulsion type cosmetic preparation of claim 1, wherein component (B) is the nonionic surfactant with an HLB of 10 to 15.

16. The oil-in-water emulsion type cosmetic preparation of claim 1, wherein component (C) comprises one or more water-base ingredients selected from the group consisting of water; lower alcohols including ethyl alcohol and butyl alcohol; glycols including propylene glycol, 1,3-butylene glycol, dipropylene glycol and polyethylene glycol; glycerols including glycerin, diglycerin and polyglycerin; and aqueous extracts from plants including aloe vera, witch hazel, hamamelis, cucumber, lemon, lavender and rose.

17. The oil-in-water emulsion type cosmetic preparation of claim 1, wherein component (A) with an OHV of 35 to 110 is: (a) the ester compound of formula (I) and/or formula (II), (b) the polycondensate of erythritol and/or erythritol condensate, fatty acid and polycarboxylic acid, or a mixture of (a) and (b);
component (B) is a polyoxyethylene sorbitan fatty acid ester with an HLB of 10 to 15; and
component (C) comprises one or more water-base ingredients selected from the group consisting of water, ethyl alcohol, 1,3-butylene glycol, dipropylene glycol and glycerin.

18. The oil-in-water emulsion type cosmetic preparation of claim 17, wherein component (A) is an ester compound of erythritol and 2-ethylhexanoic acid, an ester compound of erythritol and isostearic acid, a polycondensate of erythritol, isostearic acid and succinic acid, or a mixture thereof; and
component (B) is polyoxyethylene sorbitan monostearate, polyoxyethylene sorbitan monooleate or polyoxyethylene sorbitan trioleate.

19. The oil-in-water emulsion type cosmetic preparation of any one of claims 1 to 18, wherein component (A) is contained in an amount of 0.1 to 30% by mass, component (B) is contained in an amount of 0.01 to 10% by mass and component (C) is contained in an amount of 40 to 95% by mass.

20. The oil-in-water emulsion type cosmetic preparation of claim 19, wherein component (A) is contained in an amount of 1 to 10% by mass, component (B) is contained in an amount of 0.1 to 2% by mass and component (C) is contained in an amount of 70 to 90% by mass.
